# EUROPÄISCHE PATENTSCHRIFT

(11) **EP 1 545 357 B1**
(45) Veröffentlichungstag und Bekanntmachung des Hinweises auf die Patenterteilung: **15.03.2006**
(21) Anmeldenummer: 03797295.7
(22) Anmeldetag: 11.09.2003
(51) Int. Cl.: A61B 17/80, A61B 17/86

(54) **SYSTEM FÜR DIE OSTEOSYNTHESE**
SYSTEM FOR OSTEOSYNTHESIS
SYSTÈME POUR L'OSTÉOSYNTHÈSE

(30) Priorität: 17.09.2002 DE 10243791
(43) Veröffentlichungstag der Anmeldung: 29.06.2005
(73) Patentinhaber: DePuy Spine Sàrl, 2400 Le Locle (CH)
(72) Erfinder: SCHÄFER, Bernd, 6315 Oberägeri (CH)
(74) Vertreter: Steimle, Josef
(86) Internationale Anmeldenummer: PCT/EP2003/010104
(87) Internationale Veröffentlichungsnummer: WO 2004/026159

(56) Entgegenhaltungen:
- DE-A- 4 409 833
- DE-A- 19 950 270
- US-A- 1 151 861
- US-A1- 2001 035 075
- US-B1- 6 306 136

## Beschreibung

Die Erfindung betrifft ein System für die Osteosynthese gemäß dem Oberbegriff des Anspruchs 1, wie es zum Beispiel aus der DE-A-199 50 270 bekannt ist. Knochenplatten werden zum Beispiel an Wirbeln befestigt, um die Wirbel zu orientieren und zu stabilisieren. Hierfür werden die einzelnen Knochenplatten über Stäbe miteinander verbunden, wobei die Stäbe an den Knochenplatten befestigt, insbesondere festgeklemmt werden.

Zum Befestigen der Knochenplatten an den Wirbeln werden Knochenschrauben verwendet, die die Knochenplatten durchdringen und in den Wirbel eingeschraubt werden. Die Knochenplatte wird in der Regel mittels des Schraubenkopfes festgehalten (DE-A-44 09 833).

Aus der US 5,275,601 ist ebenfalls eine Knochenschraube bekannt, die mit ihrem, eine Riffelung aufweisenden, konischen Schraubenkopf in einer Aufnahmeöffnung einer Platte ruht. Die Platte wird mit diesen Schrauben am Knochen befestigt.

Beiden Knochenplatten liegt die Aufgabe zugrunde, die am Knochen wirkenden Kräfte optimal aufnehmen zu können, so dass sie entweder auf einen benachbarten Knochen oder Knochenteil oder auf Korrektur- oder Fixierstäbe übertragen werden können. Hierfür ist es unbedingt erforderlich, dass der Schraubenkopf optimal in der Aufnahmeöffnung der Knochenplatte liegt, was bedeutet, dass ein Spiel zwischen Schraubenkopf und Aufnahmeöffnung auf jeden Fall vermieden werden muss. Eine Sicherung gegen unbeabsichtigtes Herausdrehen wird bereits durch die Riffelung, die sich am Innenumfang der Aufnahmeöffnung und/oder am Außenumfang des Schraubenkopfes befindet, geschaffen.

Der Erfindung liegt die Aufgabe zugrunde, ein System bereitzustellen, bei welchem eine größere Sicherheit für eine innige Verbindung, das heißt eine optimale Anlage der Außenumfangsfläche des Schraubenkopfes an der Innenumfangsfläche der Aufnahmeöffnung der Knochenplatte, besteht.

Diese Aufgabe wird mit einem System für die Osteosynthese gelöst, das die Merkmale des Anspruchs 1 aufweist.

Bei der erfindungsgemäßen Halswirbelplatte sind ebenfalls Riffelungen am Innenumfang der Aufnahmeöffnung vorgesehen, jedoch ist die Tiefe der Riffelung nicht konstant, sondern nimmt in distaler Richtung zu. Die Riffelung kann sich über die gesamte Länge der Aufnahmeöffnung erstrecken, wobei dies jedoch nicht unbedingt erforderlich ist. Der Vorteil der erfindungsgemäßen Halswirbelplatte besteht darin, dass der Schraubenkopf an der proximalen Seite der Aufnahmeöffnung über einen großen Bereich der Innenumfangsfläche formschlüssig anliegt. Dieser Bereich für die formschlüssige Anlage nimmt allmählich in distaler Richtung ab. Durch die innige Anlage des Schraubenkopfes im proximalen Bereich wird gewährleistet, dass die Knochenplatte in unmittelbarer Nachbarschaft zum Knochen optimal formschlüssig von der Knochenschraube gehalten wird. Hierdurch werden die am Knochen auftretenden Kräfte unmittelbar an der Knochenoberfläche über die Knochenschraube, das heißt über den Schraubenkopf, in die Knochenplatte eingeleitet. Der Vorteil wird darin gesehen, dass keine Biegemomente auf die Schraube wirken und diese deshalb kleiner dimensioniert werden kann. Dies ist vor allem im Bereich der Halswirbel wichtig, da dort die Wirbel kleine Abmessungen aufweisen und daher groß dimensionierte Implantate nicht verwendet werden können.

Erfindungsgemäß wird, wie bei der Innenumfangsfläche der Aufnahmeöffnung der Halswirbelplatte, an der Außenumfangsfläche des Schraubenkopfes der tragende Bereich, mit welcher der Schraubenkopf an der Innenumfangsfläche der Aufnahmeöffnung formschlüssig anliegt, vergrößert. Auf diese Weise kann auch seitens der Knochenschraube die Sicherheit für eine optimale Kraftübertragung zwischen dem Wirbel und der Halswirbelplatte erhöht werden. Die einzelnen Einschnitte der Riffelung sind bei dieser Ausführungsform im Wesentlichen kugelsegmentförmig ausgebildet, so dass der Schraubenkopf sowohl im distalen Bereich als auch im proximalen Bereich große, tragende Flächen besitzt.

Um den Sitz der Knochenschraube, insbesondere deren Formschluss zu verbessern, erweitern sich die Aufnahmeöffnungen in der Halswirbelplatte in distaler Richtung. Vorteilhaft sind die Aufnahmeöffnungen konisch oder kalottenförmig ausgebildet. Dadurch kann der Schraubenkopf in der Halswirbelplatte versenkt werden und legt sich beim Anziehen der Schraube formschlüssig an der Innenumfangsfläche der Aufnahmeöffnung an.

Mit Vorzug ist die Riffelung keilförmig ausgestaltet. Dies hat den Vorteil, dass die Keilflanken, welche in den Innenraum der Aufnahmeöffnung gerichtet sind, sich in die Außenoberfläche des Schraubenkopfes eingraben können, beziehungsweise sich mit dem Schraubenkopf verhaken können. Hierdurch wird eine Ausdrehsicherung geschaffen.

Bei einem bevorzugten Ausführungsbeispiel der Halswirbelplatte sind vier Aufnahmeöffnungen vorgesehen, die sich in den Eckbereichen der Halswirbelplatte befinden. Auf diese Weise können zwei benachbarte Halswirbel mit jeweils zwei Knochenschrauben an der Halswirbelplatte befestigt werden. Somit können nicht nur Zug- und Druckkräfte, sondern auch Torsions- uns Scherkräfte übertragen werden.

Bei einer Weiterbildung ist vorgesehen, dass in der zentralen Mitte der Halswirbelplatte eine weitere Aufnahmeöffnung vorgesehen ist. In diese weitere, zentrale Aufnahmeöffnung kann ebenfalls eine Knochenschraube eingedreht werden, so dass insbesondere bei unvollständigen Wirbeln oder schwer zugänglichen Wirbeln eine weitere Möglichkeit besteht, diesen Wirbel mit der Halswirbelplatte zu verbinden.

Mit Vorzug wird die Längsriffelung von keilförmigen, im Wesentlichen in Längsrichtung verlaufenden Einschnitten gebildet, wobei die einzelnen Einschnitte einen Abstand zueinander aufweisen.

In Umfangsrichtung gesehen, liegen zwischen den Einschnitten der Riffelung einschnittfreie Bereiche. Diese einschnittfreien Bereiche dienen ebenfalls zur formschlüssigen Anlage des Schraubenkopfes an der Innenumfangsfläche der Aufnahmeöffnung der Halswirbelplatte, wodurch zum einen das Eindrehen der Schraube erleichtert wird, das heißt, das Eindrehen der Schraube wird durch die Riffelungen an der Innenumfangsfläche der Aufnahmeöffnung bzw. an der Außenumfangsfläche des Schraubenkopfes nicht behindert, zum anderen wird der Auflagebereich vergrößert.

Vorzugsweise entspricht in Umfangsrichtung gesehen, die Länge des einschnittfreien Bereichs zwischen 0,3 und 2,0, insbesondere zwischen 0,5 und 1,0 der Länge des Einschnitts selbst. Bei kleinen bzw. schmalen einschnittfreien Bereichen kann der Schraubenkopf beim oder unmittelbar vor dem Ende des Eindrehens einfacher verformt werden, so dass er sich mit den Einschnitten bzw. mit der Riffelung in der Aufnahmeöffnung der Halswirbelplatte verhakt, und dadurch gegen ein Rückdrehen gesichert ist. Bei großen einschnittfreien Bereichen besitzt der Schraubenkopf relativ große Anlageflächen, so dass eine hohe Sicherheit für eine optimale Kraftübertragung gegeben ist.

Weitere Vorteile, Merkmale und Einzelheiten der Erfindung ergeben sich aus den Unteransprüchen und der nachfolgenden Beschreibung, in der unter Bezugnahme auf die Zeichnung besonders bevorzugte Ausführungsbeispiele im Einzelnen beschrieben sind. Dabei können die in der Zeichnung dargestellten, sowie in der Beschreibung und in den Ansprüchen erwähnten Merkmale jeweils einzeln für sich oder in beliebiger Kombination erfindungswesentlich sein.

In der Zeichnung zeigen:
- Figur 1: eine perspektivische Ansicht einer Halswirbelplatte mit eingesetzten Knochenschrauben;
- Figur 2: eine vergrößerte Wiedergabe eines Eckbereichs der Halswirbelplatte, eine Aufnahmeöffnung zeigend; und
- Figur 3: eine perspektivische Ansicht einer Knochenschraube.

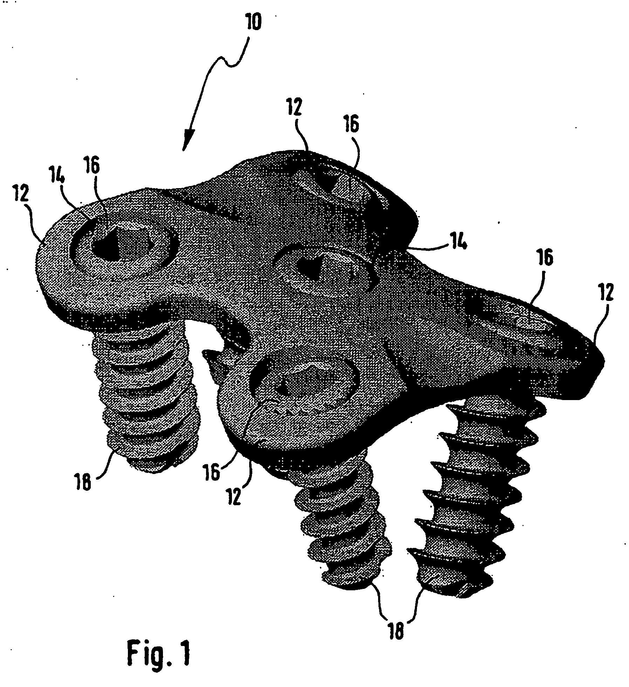

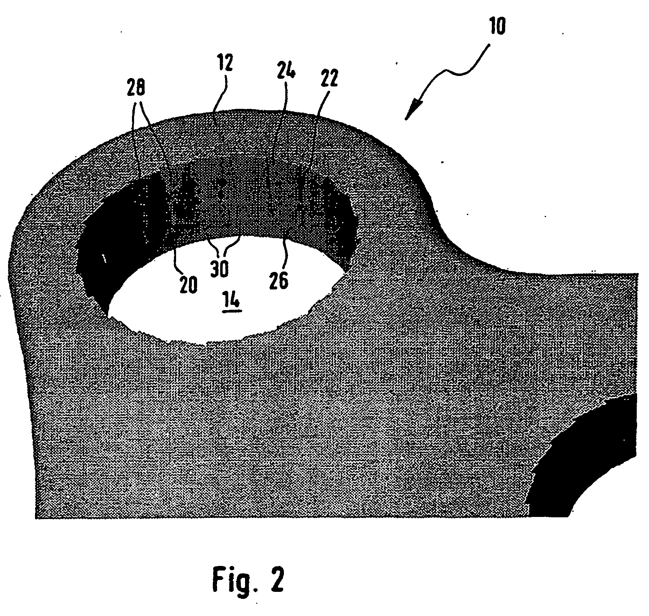

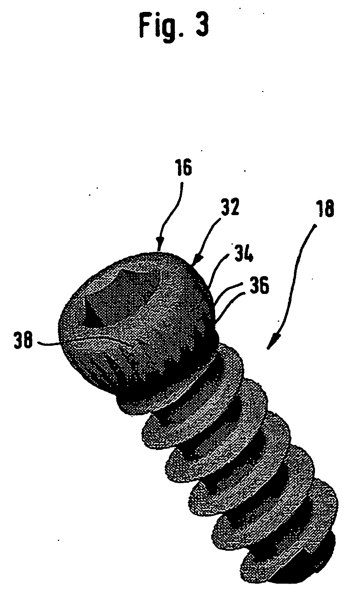

Die Figur 1 zeigt eine insgesamt mit 10 bezeichnete Halswirbelplatte, die eine im Wesentlichen H-förmige Grundform aufweist. In den Eckbereichen 12 sind Aufnahmeöffnungen 14 (siehe Figur 2) vorgesehen, die zur Aufnahme von Schraubenköpfen 16 (siehe Figur 3) von Knochenschrauben 18 dienen. Ferner weist die Halswirbelplatte 10 in ihrer zentralen Mitte eine weitere Aufnahmeöffnung 14 auf, in welcher eine weitere Knochenschraube 18 gelagert ist.

Die Halswirbelplatte 10 dient zur Verbindung zweier Halswirbel, wobei in den einen Halswirbel zwei der Knochenschrauben 18 und in den anderen Halswirbel die beiden anderen Knochenschrauben 18 eingedreht werden. Die zentrale Knochenschraube wird dann verwendet, wenn ein Halswirbel nicht vollständig ist beziehungsweise in einen Halswirbel eine der in den Eckbereichen 12 gelagerten Knochenschrauben 18 nicht eingedreht werden kann.

Wie aus Figur 2 ersichtlich, weist die Aufnahmeöffnung 14 eine Innenumfangsfläche 20 auf, die mit einer Riffelung 22, insbesondere einer Längsriffelung, versehen ist. Dabei ist deutlich erkennbar, dass sich die Riffelung 22 ausgehend vom distalen Ende 24 der Aufnahmeöffnung 20 nicht vollständig bis zum proximalen Ende 26 erstreckt, sondern lediglich über etwa 80% der Dicke der Halswirbelplatte 10. Außerdem ist erkennbar, dass sich die Tiefe der Riffelung 22 über die Höhe der Aufnahmeöffnung 14 ändert. Dabei nimmt die Tiefe vom proximalen Ende 26 in Richtung des distalen Endes 24 zu. Somit entsteht eine Riffelung 22 mit etwa keilförmigen Einschnitten 28. Ferner ist erkennbar, dass zwischen einander benachbarten Einschnitten 28 einschnittfreie Bereiche 30 liegen.

Diese einschnittfreien Bereiche 30 dienen zur formschlüssigen Anlage des Schraubenkopfes 16 und zur Kraftübertragung zwischen Schraubenkopf 16 und Halswirbelplatte 10.

In der Figur 2 ist ferner erkennbar, dass die Breite der einschnittfreien Bereiche 30 in proximaler Richtung zunimmt, so dass im Bereich des proximalen Endes 26 der Schraubenkopf 16 über den ganzen Umfang formschlüssig anliegt.

Die Figur 3 zeigt eine Knochenschraube 18, die mit einem kugelförmigen Schraubenkopf 16 versehen ist. Der Außenumfang 32 des Schraubenkopfes 16 ist mit einer Riffelung 34 versehen, die von kugelsegmentförmigen Einschnitten 36 gebildet wird. Dabei nimmt die Tiefe eines jeden Einschnitts 36 vom proximalen Ende des Schraubenkopfes 16 in Richtung des distalen Endes zunächst einmal zu und ab dem Großkreis (maximaler Durchmesser des Schraubenkopfes 16) wieder ab. Zwischen den einzelnen Einschnitten 36 befindet sich ein einschnittfreier Bereich 38, dessen Breite vom Großkreis ausgehend, in Richtung des proximalen Endes und in Richtung des distalen Endes zunimmt.

Die Riffelung 22 der Halswirbelplatte 10 sowie die Riffelungen 34 der Knochenschraube 18 sind derart ausgebildet, dass sie ein Einschrauben der Knochenschraube 18 erlauben, jedoch eine Hemmwirkung in Gegenrichtung ausüben, so dass die Knochenschraube 18 daran gehindert wird, sich durch geringfügiges Ausschrauben in der Aufnahmeöffnung 14 zu lockern.

## Patentansprüche

1. System für die Osteosynthese mit - einer Wirbelplatte (10) mit wenigstens zwei Aufnahmeöffnungen (14) für die Schraubenköpfe (18) von Knochenschrauben (16) zum Befestigen der Wirbelplatte (10) an zwei Wirbeln, wobei die Aufnahmeöffnungen (14) im distalen Bereich über ihren Umfang eine Riffelung (22), insbesondere Längsriffelung, aufweisen, wobei die Tiefe der Riffelung (22) von proximal nach distal zunimmt, und - einer Knochenschraube (18) mit einem Schraubenkopf (16) und einem ein Gewinde aufweisenden Schraubenschaft, wobei der Schraubenkopf (16) an seinem Außenumfang eine Riffelung (34), insbesondere eine Längsriffelung, aufweist, wobei die Riffelung (34) über deren Länge gesehen, eine sich ändernde Tiefe aufweist und der Schraubenkopf (16) im Wesentlichen kugelförmig ist, **dadurch gekennzeichnet dass** die - die Wirbelplatte eine Halswirbelplatte ist, und dass - die Tiefe der Riffelung (34) am Schraubenkopf (16) jeweils von den Polen in Richtung des Großkreises zunimmt.

2. System nach Anspruch 1, **dadurch gekennzeichnet, dass** die Aufnahmeöffnungen (14) sich in distaler Richtung, insbesondere konisch oder kalottenförmig, erweitern.

3. System nach einem der vorhergehenden Ansprüche, **dadurch gekennzeichnet, dass** die Riffelung (22) keilförmig ist.

4. System nach einem der vorhergehenden Ansprüche, **dadurch gekennzeichnet, dass** vier Aufnahmeöffnungen (14) vorgesehen sind, die sich in den Eckbereichen (12) der Halswirbelplatte (10) befinden.

5. System nach einem der vorhergehenden Ansprüche, **dadurch gekennzeichnet, dass** in der zentralen Mitte der Halswirbelplatte (10) eine weitere Aufnahmeöffnung (14) vorgesehen ist.

6. System.nach einem der vorhergehenden Ansprüche, **dadurch .gekennzeichnet, dass** die Riffelung (22) von keilförmigen, im Wesentlichen in Längsrichtung verlaufenden, Einschnitten (28) gebildet wird, und die einzelnen Einschnitte (28) einen Abstand zueinander aufweisen, zwischen denen insbesondere einschnittsfreie Bereiche (30) liegen.

7. System nach einem der vorhergehenden Ansprüche, **dadurch gekennzeichnet, dass** die Riffelung (34) von keilförmigen, im Wesentlichen in Längsrichtung verlaufenden, Einschnitten (36) gebildet wird, und die einzelnen Einschnitte (36) einen Abstand zueinander aufweisen.

8. System nach einem der vorhergehenden Ansprüche, **dadurch gekennzeichnet, dass** in Umfangsrichtung gesehen, zwischen den Einschnitten (36) der Riffelung (34) einschnittsfreie Bereiche (38) liegen.

9. System nach Anspruch 8, **dadurch gekennzeichnet, dass**, in Umfangsrichtung gesehen, die Länge des einschnittsfreien Bereichs (38) zwischen 0,3 und 2,0, insbesondere 0,5 bis 1,0, der Länge eines Einschnitts (36) entspricht.

## Claims

1. System for osteosynthesis, comprising:
- a vertebra plate (10) having at least two receiving openings (14) for the screw heads (18) of bone screws (16) for securing the vertebra plate (10) to two vertebrae, wherein the receiving openings (14) have a fluting (22), in particular a longitudinal fluting, about the periphery of a distal area, wherein the depth of the fluting (22) increases from the proximal to the distal, and
- a bone screw (18) having a screw head (16) and a threaded screw shank, wherein the outer periphery of the screw head (16) has a fluting (34), in particular a longitudinal fluting, wherein the fluting (34) has a varying depth along its length and the screw head (16) is substantially spherical,
**characterized in that**
- the vertebra plate (10) is a cervical, and that
- the depth of the fluting (34) at the screw head (16) increasing from each pole towards the equator.

2. System according to claim 1, **characterized in that** the receiving openings (14) widen in a distal direction, in particular in a conical or dome-shaped fashion.

3. System according to any one of the preceding claims, **characterized in that** the fluting (22) is wedge-shaped.

4. System according to any one of the preceding claims, **characterized in that** four receiving openings (14) are provided which are located in the corner areas (12) of the cervical vertebra plate (10).

5. System according to any one of the preceding claims, **characterized in that** a further receiving opening (14) is provided in the center of the cervical vertebra plate (10).

6. System according to any one of the preceding claims, **characterized in that** the fluting (22) is formed by wedge-shaped grooves (28) which extend in a substantially longitudinal direction, and the individual grooves (28) have a mutual separations with areas (30) without grooves therebetween.

7. System according to any one of the preceding claims, **characterized in that** the fluting (34) is formed by wedge-shaped grooves (36) which extend substantially in a longitudinal direction, and the individual grooves (36) being separated from each other.

8. System according to any one of the preceding claims, **characterized in that**, viewed in the peripheral direction, areas (38) without grooves are provided between the grooves (36) of the fluting (34).

9. System according to claim 8, **characterized in that**, viewed in the peripheral direction, the length of the area (38) without grooves corresponds to between 0.3 and 2.0, in particular 0.5 to 1.0 times the length of a groove (36).

## Revendications

1. Système pour l'ostéosynthèse comprenant
- une plaque d'ostéosynthèse vertébrale (10) avec au moins deux orifices (14) recevant les têtes (18) de vis d'ostéosynthèse (16) destinées à solidariser la plaque d'ostéosynthèse vertébrale (10) à deux vertèbres, les orifices (14) présentant sur la région distale de leur pourtour un striage (22), notamment un striage longitudinal, la profondeur dudit striage (22) augmentant de la région proximale vers la région distale, et
- une vis d'ostéosynthèse (18) avec une tête (16) et un corps fileté, la tête de vis (16) présentant sur son pourtour externe un striage (34), notamment un striage longitudinal, ledit striage (34) présentant une profondeur variable dans le sens de la longueur, et la tête de vis (16) affectant une forme sensiblement sphérique,
**caractérisé en ce que**
- la plaque d'ostéosynthèse vertébrale est une plaque cervicale et **en ce que**
- la profondeur du striage (34) au niveau de la tête de vis (16) augmente des pôles en direction du grand cercle.

2. Système selon la revendication 1, **caractérisé en ce que** les orifices (14) s'élargissent en direction distale, notamment selon une forme conique ou une forme de calotte.

3. Système selon l'une quelconque des revendications précédentes, **caractérisé en ce que** le striage (22) est en forme de coin.

4. Système selon l'une quelconque des revendications précédentes, **caractérisé en ce qu'**il comporte quatre orifices (14) situés dans les coins (12) de la plaque cervicale (10).

5. Système selon l'une quelconque des revendications, **caractérisé en ce qu'**un orifice (14) supplémentaire est prévu au centre de la plaque cervicale (10).

6. Système selon l'une quelconque des revendications précédentes, **caractérisé en ce que** le striage (22) est formé par des entailles (28) en forme de coin s'étendant sensiblement dans la direction longitudinale et **en ce que** les différentes entailles (28) sont ménagées à distance les unes des autres et sont séparées notamment par des régions (30) exemptes d'entaille.

7. Système selon l'une quelconque des revendications précédentes, **caractérisé en ce que** le striage (34) est formé par des entailles (36) en forme de coin s'étendant sensiblement dans la direction longitudinale et **en ce que** les différentes entailles (36) sont ménagées à distance les unes des autres.

8. Système selon l'une quelconque des revendications précédentes, **caractérisé en ce que** des régions (38) exemptes d'entaille sont présentes entre les entailles (36) du striage (34), vu dans la direction circonférentielle.

9. Système selon la revendication 8, **caractérisé en ce que**, vu dans la direction circonférentielle, la longueur de la région (38) exempte d'entaille est comprise entre 0,3 et 2,0 fois, notamment entre 0,5 et 1,0 fois la longueur d'une entaille (36).
